# EUROPÄISCHE PATENTSCHRIFT

(11) **EP 3 917 592 B1**
(45) Veröffentlichungstag und Bekanntmachung des Hinweises auf die Patenterteilung: **23.04.2025**
(21) Anmeldenummer: 20703951.2
(22) Anmeldetag: 30.01.2020
(51) Int. Cl.: A61M 1/16, A61M 1/36

(54) **EXTRAKORPORALE BLUTBEHANDLUNGSVORRICHTUNG MIT FUNKTIONSÜBERWACHUNGSSYSTEM**
EXTRACORPOREAL BLOOD-TREATMENT DEVICE WITH FUNCTION-MONITORING SYSTEM
DISPOSITIF DE TRAITEMENT EXTRACORPOREL DU SANG AVEC SYSTÈME DE SURVEILLANCE DU FONCTIONNEMENT

(30) Priorität: 31.01.2019 DE 102019000732
(43) Veröffentlichungstag der Anmeldung: 08.12.2021
(73) Patentinhaber: Pulsion Medical Systems SE, 85622 Feldkirchen (DE)
(72) Erfinder: KAMMERZELL, Sergej, 81827 München (DE); KONRAD, Mark, 81541 München (DE)
(74) Vertreter: Kehl, Ascherl, Liebhoff & Ettmayr Patentanwälte Partnerschaft mbB
(86) Internationale Anmeldenummer: PCT/EP2020/052336
(87) Internationale Veröffentlichungsnummer: WO 2020/157224

(56) Entgegenhaltungen:
- WO-A1-01/30237
- US-A1- 2012 298 581
- US-B1- 6 187 199

## Beschreibung

### Technisches Gebiet

Die vorliegende Erfindung betrifft eine extrakorporale Blutbehandlungsvorrichtung mit einem Funktionsüberwachungssystem gemäß Anspruch 1, sowie ein Computersystem und ein computerlesbares Speichermedium zur Ausführung eines Verfahrens zur Überwachung des Funktionszustandes einer extrakorporalen Blutbehandlungsvorrichtung gemäß den Ansprüchen 13 und 14.

### Stand der Technik

Für eine Reihe von Krankheitsbildern in Patienten ist eine extrakorporale Blutbehandlung indiziert, beispielsweise die Hämodialyse oder Hämofiltration bei Nieren- und Lebererkrankungen sowie die extrakorporale Decarboxylierung / Membranoxygenierung (ECMO) bei der Therapie von schweren Lungenerkrankungen (akutes Atemnotsyndrom, ARDS) und von Herzerkrankungen (Myokardinfarkt, schwere Rhythmusstörungen). Die Hämodialyse im Rahmen der Nierenersatztherapie beinhaltet die extrakorporale Reinigung des Blutes eines Patienten von Blutinhaltsstoffen, die bei einem Gesunden über die Nieren ausgeschieden werden. Diese sogenannten "harnpflichtigen" Blutinhaltsstoffe werden bei der Hämodialyse diffusiv über eine semipermeable Membran aus dem Blut in eine Dialysierflüssigkeit transportiert. Im Unterschied dazu findet bei der Hämofiltration ein konvektiver Stofftransport über eine semipermeable Filtermembran statt, wobei ein Druckgradient an der Membran die treibende Kraft für den Stofftransport ist. Die Hämodiafiltration vereinigt beide Prinzipien, indem kleinere Moleküle über Diffusion und größere Substanzen über Konvektion aus dem Blut entfernt werden und damit in der Regel die Gesamteliminationsrate harnpflichtiger Substanzen gesteigert wird. Die extrakorporale Membranoxygenierung im Rahmen der Therapie schwerer Lungenerkrankungen gehört zur Standardtherapie für die Stabilisierung des Blutkreislaufs und/oder der Atemfunktion von Patienten mit ARDS und lebensbedrohlicher Hypoxämie. In der ECMO- Vorrichtung wird das Blut mittels einer Pumpe über einen Membranoxygenator perfundiert. Dieser beherbergt eine semipermeable Membran, über welche der Gasaustausch stattfindet, indem auf einer Seite das Patientenblut fließt und auf der anderen Seite Sauerstoff eingeleitet wird. Die Pumpe produziert dabei einen Blutfluss von 2 bis 6 l/min, sodass eine effiziente Oxygenierung und Kohlendioxidelimination möglich ist.

Bei der Überwachung einer derartigen Blutbehandlung ist die Erfassung der Blutbehandlungsleistung von Bedeutung. Bei einer Blutreinigung, beispielsweise mittels Hämodialyse oder Hämofiltration im Rahmen einer Nieren- oder Leberersatztherapie, wird die Blutreinigungsleistung gewöhnlich anhand bestimmter Parameter überwacht: beispielsweise gibt die im Rahmen der Hämodialyse bestimmte "Clearance" den hypothetischen Teil des Blutstroms an, welcher pro Minute vollständig von einer bestimmten Substanz befreit wird. Die Blutbehandlungsleistung während der Hämodialyse wird aber durch den Wert der Clearance nur zum Teil bestimmt. Allgemein hängt die Blutbehandlungsleistung von Vorrichtungen, bei denen die Blutbehandlung über eine Membran erfolgt, von einer Vielzahl von Faktoren ab, z.B. der Membranfläche in der Behandlungsvorrichtung, der Permeabilität der Membran, der Flussrate des Blutes innerhalb der Behandlungsvorrichtung und/oder der Austauschflüssigkeit, z.B. der Dialysierflüssigkeit in einem Hämodialysator. Dabei nimmt die Effizienz der Blutbehandlung im Zeitverlauf ab, z.B. durch Verstopfung der Membran der Blutbehandlungsvorrichtung. Beispielsweise kann die maximale Gebrauchsdauer einer aus Hohlfasern bestehenden Hämofiltrations-Membran zur Beseitigung von Flüssigkeit und Toxinen in der Nierenersatztherapie durch die Ablagerung kleiner Blutgerinnsel (Thromben) in den Lumina der Fasern lediglich 15-40 Stunden betragen. Auch in der Membran eines Oxygenators kann es aufgrund von Antikörper- (Blutprotein) Ablagerungen an der Gas-/Blutmembran zu einer Effizienzabnahme kommen, da die Gas-/ Blutmembran verstopft und damit die Gasaustauschkapazität der Membran verringert wird. Die verringerte Effizienz führt zu einer Reduktion der Sauerstoffversorgung des Blutes auf unter 100%. Die Thrombose der Membran, über welche die Blutbehandlung vorgenommen wird, tritt in der Regel langsam über einen variablen Zeitraum von einigen Stunden auf. In herkömmlichen Blutbehandlungsvorrichtungen wird eine Funktionsstörung über die Veränderung der systemischen Druckverhältnisse, über den Abfall der Behandlungsleistung sowie auch optisch über die Erfassung der Gerinnselbildung (Thromben) in der Vorrichtung mehr oder weniger frühzeitig erfasst. Die Bestimmung sogenannter D-Dimere, welche durch die Plasmin-vermittelte Proteolyse von vernetztem Fibrin in einem Thrombus entstehen, stellt eine sensitive Technik zur frühzeitigen Erkennung einer Funktionsstörung der Membran einer Blutbehandlungsvorrichtung dar. Nachteilig erfordert die Bestimmung dieser Degradationsprodukte die wiederholte Entnahme von Blut aus dem System und ist damit technisch aufwendig und teuer.

Es besteht daher ein Bedarf, die Effizienz der Blutbehandlung auf der Membranebene, beispielsweise der Membran einer Vorrichtung zur extrakorporalen Membranoxygenierung, effizient und kontinuierlich zu überwachen, um zum frühestmöglichen Zeitpunkt das Behandlungsverfahren entsprechend anzupassen, die Lebensdauer der Membran zu maximieren und eventuelle Schäden vom Patienten abzuwenden.

Aus US 2012/0298581 A1 ist eine extrakorporale Blutbehandlungsvorrichtung sowie ein Verfahren zur Überwachung des Funktionszustandes der Blutbehandlungsvorrichtung bekannt, wobei ein Temperaturbolus von einem Wärmetauscher stromaufwärts der Dialysierflüssigkeitskammer des Dialysators erzeugt wird. US 6,187,199 offenbart ein Verfahren zur hämodynamischen Überwachung, wobei ein Temperaturbolus stromaufwärts von der Dialysierflüssigkeitskammer des Dialysators erzeugt wird. In WO 01/30237 A1 ist ein System zur Durchführung transpulmonaler Thermodilution einschließlich grundlegender Gleichungen zur Berechnung hieraus ermittelbarer physiologischer Parameter beschrieben.

### Kurze Beschreibung der Erfindung

In einem ersten Aspekt bezieht sich die Erfindung auf eine extrakorporale Blutbehandlungsvorrichtung mit Funktionsüberwachungssystem. Bei der extrakorporalen Blutbehandlungsvorrichtung (EKBV) kann es sich beispielsweise um eine Vorrichtung zur Hämodialyse, eine Vorrichtung zur Leberunterstützung oder um eine extrakorporale Vorrichtung zur Decarboxylierung und oder zum Membranoxygenierung handeln. Die EKBV weist zur Verbindung mit dem Gefäßsystem eines Patienten einen zuführenden und einen abführenden Schenkel auf, wobei der zuführende Schenkel der Blutbehandlungsvorrichtung Blut aus dem Gefäßsystem des Patienten zuführt; das behandelte Blut wird über den abführenden Schenkel wieder in das Gefäßsystem des Patienten eingeleitet. Beispielsweise leitet bei einer rein venösen extrakorporalen Membranoxygenierung (veno-venöse ECMO, vvECMO) ein in der unteren Hohlvene liegender Drainagekatheter venöses Blut zum Membranoxygenator, und ein in der Jugularvene oder der oberen Hohlvene liegender Katheter führt sauerstoffreiches Blut zum rechten Vorhof (oder umgekehrt). Die erfindungsgemäße EKBV weist in einem ersten Kreislauf mindestens eine zwischen dem zuführenden und dem abführenden Schenkel angeordnete erste Pumpe zur Bewegung von Patientenblut auf. Die Pumpe befördert das Blut über die entsprechende Behandlungseinheit, beispielsweise die Dialysiermembran oder die Decarboxylator-/Oxygenatormembran. Die Pumpe kann dabei jede aus dem Stand der Technik bekannte Art von Pumpe sein, beispielsweise eine Rollerpumpe oder eine Zentrifugalpumpe; bevorzugt ist die Pumpe eine Zentrifugalpumpe, da dadurch die Beschädigung korpuskulärer Bestandteile des Blutes und konsekutiv die Freisetzung gerinnungsaktiver Substanzen vermindert wird. Die Pumpe kann das Patientenblut mit einer konstanten und/oder variablen Geschwindigkeit durch den ersten Kreislauf bewegen. Weiterhin weist die EKBV in einem zweiten, mit dem ersten Kreislauf der EKBV über einen Wärmetauscher thermisch verbundenen, flüssigkeitsgefüllten Kreislauf Temperaturbeeinflussungsmittel auf. Unter einem Wärmetauscher (Wärmeübertrager) wird eine Vorrichtung verstanden, welche thermische Energie von einem Stoffstrom auf einen anderen überträgt; der erfindungsgemäße Wärmetauscher überträgt die thermische Energie der im zweiten Kreislauf zirkulierenden Flüssigkeit auf die durch die wärmedurchlässige Wand des Wärmetauschers davon getrennte Flüssigkeit des ersten Kreislaufs (indirekte Wärmeübertragung). Der erfindungsgemäße Wärmetauscher kann dabei mit ungerichteter Strömungsrichtung ("diffuse flow"), nach dem Gegenstrom- oder dem Gleichstromprinzip arbeiten; bevorzugt arbeitet der Wärmetauscher mit ungerichteter Strömungsrichtung oder nach dem Gegenstromprinzip. Die erfindungsgemäßen Temperaturbeeinflussungsmittel sind dazu eingerichtet, eine Temperatur der Flüssigkeit im zweiten Kreislauf zu beeinflussen, um über Wärmefluss (über den Wärmetauscher) eine Temperatur der Flüssigkeit im ersten Kreislauf zu beeinflussen. Unter dem Begriff "Beeinflussung" wird dabei sowohl eine (signifikante) Änderung der Temperatur, beispielsweise von Raumtemperatur auf 0 °C, als auch eine Modulation der Temperatur verstanden, beispielsweise eine Temperaturabweichung von 5° -10 °C um einen Mittelwert. Insbesondere erzeugen die Temperaturbeeinflussungsmittel im zweiten Kreislauf eine Temperaturänderung, besonders bevorzugt einen Temperaturbolus, d. h. eine wandernde Temperaturabweichung mit schnellem Anstieg und Abfall, um die Temperaturänderung in minimaler Zeit auf das Niveau der Maximaltemperaturänderung zu bringen. Die Temperaturbeeinflussungsmittel können dabei als aus dem Stand der Technik bekannte Mittel zum Beeinflussen thermischer Energie ausgebildet sein, beispielsweise als Wärmequelle oder als Kühlkörper in Form eines Peltier-Elements oder eines entsprechend temperierten und/oder temperierbaren Wasserbades.

Das erfindungsgemäße Funktionsüberwachungssystem weist einen im zweiten Kreislauf stromaufwärts des Wärmetauschers angeordneten Temperatursensor TS2_{auf} und einen im zweiten Kreislauf stromabwärts des Wärmetauschers angeordneten Temperatursensor TS2_{ab} auf. Ferner weist das Funktionsüberwachungssystem einen im abführenden Schenkel des ersten Kreislaufs der EKBV stromabwärts des Wärmetauschers angeordneten Temperatursensor TS1_{ab} auf. Die Begriffe "stromabwärts" und "stromaufwärts" beziehen sich dabei auf die jeweiligen Flussrichtungen des ersten und zweiten Kreislaufs im Wärmetauscher. Entsprechend der Anordnung der EKBV (Flussrichtung des Patientenbluts im ersten Kreislauf) und der Arbeitsweise des Wärmetauschers (Gleichstrom-, Gegenstromprinzip, ungerichteter Stromfluss) können sich unterschiedliche Sensoranordnungen ergeben: wenn ein z.B. zylindrischer Wärmetauscher im Gegenstromprinzip arbeitet, können die stromabwärts angeordneten Temperatursensoren des ersten und zweiten Kreislaufs TS1_{ab} und TS2_{ab} bezüglich des Wärmetauschers weiter voneinander entfernt liegen (z.B. an entgegengesetzten Enden des Wärmetauschers); wenn der Wärmetauscher im Gleichstromprinzip arbeitet, können die stromabwärts angeordneten Temperatursensoren des ersten und zweiten Kreislaufs TS1_{ab} und TS2_{ab} bezüglich des Wärmetauschers nah beieinander liegen (z.B. an demselben Ende des Wärmetauschers). Die vorgesehenen Temperatursensoren können vorteilhaft wie bekannte, in Dilutionsmessverfahren eingesetzte Sensoren ausgeführt sein. Für die Temperaturmessung ist insbesondere ein Platinwiderstandssensor geeignet, daneben sind jedoch auch andere Thermowiderstände oder Thermoelemente zweckgemäß. Ein mit den Temperatursensoren (TS2_{auf}, TS2_{ab} , TS1_{ab}) und den Temperaturbeeinflussungsmitteln verbundenes und zum Funktionsüberwachungssystem gehörendes Computersystem ist dazu ausgelegt, mittels der Temperaturbeeinflussungsmittel einen Temperaturbolus im ersten Kreislauf der EBKV zu veranlassen, die an den Temperatursensoren (TS2_{auf}, TS2_{ab} , TS1_{ab}) erfassten Temperaturen T2_{auf}, T2_{ab}, T1_{ab} jeweils als Funktion der Zeit aufzuzeichnen und entsprechend Thermodilutionskurven (TDK) zu ermitteln und auszuwerten. Computerprogramme zur Durchführung von Auswerteschritten einer Thermodilution sind dabei *per se* aus dem Stand der Technik bekannt (z.B. aus der deutschen Offenlegungsschrift DE 4 214 402 A1). Das erfindungsgemäße Computersystem ist weiterhin dazu ausgelegt, die TDK2_{ab} und die TDK1_{ab} zueinander in Beziehung zu setzen und aus der Beziehung der TDK2_{ab} und TDK1_{ab} einen Indikator der EKBV-Funktion zu bestimmen.

In einer weiteren Ausführungsform kann das Computersystem dazu ausgelegt sein, die TDK2_{auf} und die TDK1_{ab} zueinander in Beziehung zu setzen und aus der Beziehung der TDK2_{auf} und TDK1_{ab} einen weiteren Indikator der EKBV-Funktion zu bestimmen.

In einer vorteilhaften Weiterbildung kann das Funktionsüberwachungssystem weiterhin einen im zuführenden Schenkel des ersten Kreislaufs der EKBV stromaufwärts des Wärmetauschers angeordneten Temperatursensor (TS1_{auf}) aufweisen, wobei der Indikator der EKBV-Funktion mittels eines Korrekturfaktors aus der Beziehung der TDK2_{auf} und der von Temperatursensor TS1_{auf} erfassten Temperatur T1_{auf} oder gegebenenfalls einer davon abgeleiteten Größe, z.B. der TDK1_{auf}, korrigiert wird.

Der von den Temperaturbeeinflussungsmitteln im ersten Kreislauf erzeugte Temperaturänderung, insbesondere ein Temperaturbolus, kann aus der Differenz der TDK2_{auf} und der TDK2_{ab} abgeschätzt werden und reflektiert diejenige Wärmemenge, die über den Wärmetauscher (Wärmeübertrager) aus dem zweiten in den ersten Kreislauf der EKBV und schließlich in den Patientenkreislauf gelangen kann. Bei einem hohen Temperaturgradienten zwischen der Bolus-Temperatur (T2_{auf} z.B. 10°C) und der Bluttemperatur im zuführenden Schenkel (T1_{auf} z.B. 38°C) ist die vom zweiten Kreislauf an den ersten Kreislauf abgegebene Wärmemenge groß, und die an TS1_{ab} gemessene Maximaltemperatur (bzw. die "area under the curve" (AUC) der TDK1_{ab}) entspricht weitgehend dem an TS2_{ab} gemessenen Wert (bzw. der AUC der TDK2_{ab}). Bei einer Funktionsstörung der EKBV, beispielsweise durch Verringerung der Austauschfläche der Oxygenatormembran infolge von lokaler Blutgerinnung ("membrane clotting") findet eine geringere Wärmeabgabe über die Membran statt: selbst bei einem hohen Temperaturgradienten gelangt bei beeinträchtigter Funktion ("Verstopfung" der Membran durch Blutgerinnsel) nur eine geringe Wärmemenge an den im abführenden Schenkel des ersten Kreislaufs der EKBV lokalisierten Temperatursensor TS1_{ab}, die dort gemessene Maximaltemperatur (bzw. die AUC der TDK1_{ab}) kann kleiner als der an Temperatursensor TS2_{ab} gemessene Wert sein. Bei unbeeinträchtigter Funktion (freie Membranfläche) wird bei einem hohen Temperaturgradienten zwischen dem ersten und zweiten Kreislauf eine hohe Wärmemenge abgegeben und diese im ersten Kreislauf über Temperatursensor TS1_{ab} weitergeleitet, d. h. die an TS1_{ab} gemessene Maximaltemperatur (bzw. die AUC der TDK1_{ab}) entspricht dem an TS2_{ab} gemessenen Wert (bzw. der AUC der TDK2_{ab}).

Die gegebenenfalls zusätzliche Berücksichtigung eines Korrekturfaktors aus der Beziehung der TDK2_{auf} und der von Temperatursensor TS1_{auf} erfassten Temperatur T1_{auf} erlaubt eine noch genauere Bestimmung des Indikators. Beispielsweise kann das Computersystem die von Temperatursensor TS1_{auf} erfasste Temperatur T1_{auf} als Funktion der Zeit aufzeichnen und entsprechend die Thermodilutionskurve TDK1_{auf} generieren. Aus dem Verhältnis TDK2_{auf}/TDK1_{auf} kann der anfängliche Temperaturgradient zwischen den beiden Kreisläufen abgeleitet werden und zur Korrektur der Beziehung von TDK2_{ab} und TDK1_{ab} dienen. Weiterhin kann über die Beziehung des vom zusätzlichen Temperatursensor TS1_{auf} gemessenen Temperaturwerts in Zusammenhang mit der TDK1_{ab} eine der extrakorporalen Blutbehandlungsvorrichtung zuzuordnende Temperaturabweichung bestimmen werden (als Maß der Rezirkulation).

Der aus der Beziehung der TDK2_{ab} und TDK1_{ab} bestimmte Indikator der EKBV-Funktion, bzw. der aus der Beziehung der TDK2_{auf} und TDK1_{ab} bestimmte weitere Indikator der EKBV-Funktion, dient zur zuverlässigen Ermittlung des Funktionszustandes der in der Blutbehandlungsvorrichtung angeordneten Funktionseinheit, nämlich der Membran, über welche die Behandlung stattfindet (z.B. Dialysiermembran, Filtrationsmembran, Decarboxylator-/Oxygenatormembran). Beispielsweise kann anhand einer einfachen Verhältnis-Relation TDK2_{ab}/TDK1_{ab} der Funktionszustand bestimmt werden, eine Verschiebung des Verhältnisses in Richtung TDK2_{ab} indiziert eine Störung der Membranfunktion, wobei die Größe der Verschiebung des Verhältnisses das Ausmaß der Störung der Membranfunktion indiziert. Vorteilhaft können mittels des erfindungsgemäßen Funktionsüberwachungssystems alle Messungen automatisiert im Gerät durchgeführt werden, es bedarf keiner zusätzlichen Funktionseinheiten oder Verfahrensschritte. Zudem ist das System im Vergleich zu herkömmlichen Überwachungsmethoden mittels Druck- oder Flow-Messung sensitiv gegenüber kleinen Veränderungen der EKBV-Funktion, z.B. Funktionseinschränkungen der Oxygenatormembran in einer ECMO.

In einer weiteren Implementierung der erfindungsgemäßen Vorrichtung kann das Computersystem dazu ausgelegt sein, mindestens eine zweite, mit den Temperaturbeeinflussungsmitteln verbundene Pumpe im zweiten Kreislauf anzusteuern, derart, dass die Pumpengeschwindigkeit zu Erzeugung einer weitgehend steilen Temperaturdifferenz angepasst ist; die Temperaturdifferenz bezieht sich insbesondere auf die Differenz zwischen der Temperatur der Flüssigkeiten im ersten und zweiten Kreislauf im Bereich des Wärmetauschers. Insbesondere bevorzugt kann die zweite Pumpe im zweiten Kreislauf stromaufwärts des Wärmetauschers angeordnet sein. Vorzugsweise kann die zweite Pumpe im zweiten Kreislauf während der Wärmeübertragung mit einer höheren Geschwindigkeit laufen als die erste Pumpe im ersten Kreislauf. Beispielsweise kann das erfindungsgemäße Computersystem dazu ausgelegt sein, Daten der ersten Pumpe im ersten Kreislauf zu erfassen, z.B. eine eingestellte Geschwindigkeit oder den aus der eingestellten Geschwindigkeit resultierenden Flow (z.B. mittels eines im ersten Kreislauf angeordneten Flow-Sensors). Das Verhältnis der entsprechenden Pumpen-Geschwindigkeiten zueinander kann zur Funktionsüber-wachung der EKBV Membran für die jeweilige Messung vorteilhaft gleich sein, sodass eine automatisierte Messung möglich ist.

In einer vorteilhaften Weiterbildung können die Temperaturbeeinflussungsmittel im ersten Kreislauf der EKBV einen Temperaturbolus erzeugen. Unter einem Temperaturbolus wird dabei eine relativ zur Temperatur des ersten Kreislaufs/Blutkreislaufs des Patienten abweichende Temperatur verstanden, die sich durch einen schnellen Anstieg und einen schnellen Abfall auszeichnet. Bei der Temperaturabweichung kann es sich um den Eintrag von Wärme oder Kälte handeln. Die in einer herkömmlichen EKBV verwendete Wärmeeinheit hält die Bluttemperatur des Patienten während der Behandlung weitgehend konstant bei 37-39°C; durch Ansteuern durch das erfindungsgemäße Computersystem mindestens einer weiteren Einheit, z.B. einer Kühleinheit der Temperaturbeeinflussungsmittel, kann zeitlich begrenzt eine steile Temperaturdifferenz für einen Kältebolus erzeugt werden. Die so ausgebildete EKBV kann auch zur Durchführung von Thermodilutionsmessungen im Patienten verwendet werden, beispielsweise zur Bestimmung hämodynamischer/kardiovaskulärer Parameter, ohne dass die Notwendigkeit besteht, an einer weiteren Stelle des Gefäßsystems des Patienten einen Temperaturbolus zu applizieren.

In einer weiteren Ausführungsform können die Temperaturbeeinflussungsmittel stromabwärts der ersten Pumpe angeordnet sein. In dieser Ausführungsform kann das Funktionsüberwachungssystem auf einfache Weise mit einer Blutbehandlungsvorrichtung verbunden werden.

In einer bevorzugten Implementierung der erfindungsgemäßen Vorrichtung können die Temperaturbeeinflussungsmittel Schaltmittel zum Schalten zwischen mindestens zwei Temperaturen umfassen. Wenn die Temperaturbeeinflussungsmittel beispielsweise als Wasserbad mit Behältern unterschiedlicher Temperatur ausgebildet sind, kann mittels der Schaltmittel der Behälter mit der gewünschten Temperatur dem zweiten Kreislauf zugeschaltet werden. Alternativ können die Schaltmittel z.B. bei als Peltier-Element ausgebildeten Temperaturbeeinflussungsmitteln die Zuführung des Mittels bzw. des dadurch mit thermischer Energie beaufschlagten zweiten Kreislaufs zum Wärmetauscher schalten, gegebenenfalls damit auch die Verbindung zum ersten Kreislauf. Insbesondere bevorzugt können die Schaltmittel auch dazu dienen, die Temperaturbeeinflussungsmittel ein- bzw. auszuschalten. In einer besonders vorteilhaften Weiterbildung können die Schaltmittel zwischen mindestens zwei unterschiedlich temperierten Flüssigkeitsreservoirs schalten; beispielsweise zwischen einem Reservoir mit einer der Temperatur im ersten Kreislauf entsprechenden Temperatur und einem Reservoir mit einer mindestens um 10 °C davon abweichenden Temperatur.

In einer weiteren Ausführungsform der erfindungsgemäßen Vorrichtung kann die EKBV eine Vorrichtung zur extrakorporalen Membranoxygenierung (ECMO) sein. In einer besonders bevorzugten Weiterbildung der erfindungsgemäßen Blutbehandlungsvorrichtung können die Temperaturbeeinflussungsmittel im Bereich des Oxygenators der ECMO angeordnet sein. Der Ausdruck "im Bereich" bezieht sich dabei auf enge räumliche Beziehung; dies ist vorteilhaft, um die Abmessungen der ECMO möglichst gering zu halten. Insbesondere bei der ECMO ist eine kontinuierliche Funktionsüberwachung der Oxygenatormembran indiziert, da bei den schwerkranken Patienten eine Funktionsstörung der Membran durch Thrombenbildung nicht nur zu einem verminderten Gasaustausch, sondern auch zu einer der Membranfunktionsstörung zeitlich nachgeordneten Gerinnungsstörung im Patienten führen kann (Dornia et al., 2015).

In einer bevorzugten Implementierung der erfindungsgemäßen Vorrichtung können die Temperaturbeeinflussungsmittel extern an eine Heizeinheit der EKBV angeschlossen werden. Beispielsweise können als Peltier- Element ausgebildete Temperaturbeeinflussungsmittel an entsprechende Zu- oder Abführungen der Heizeinheit der EKBV angeschlossen werden. Vorteilhaft kann so die Nachrüstung einer EKBV ohne Funktionsüberwachungssystem auf einfache Art und Weise vorgenommen werden.

In einem ersten Schritt eines Verfahrens zur Überwachung des Funktionszustandes einer erfindungsgemäßen extrakorporalen Blutbehandlungsvorrichtung wird im zweiten Kreislauf der EKBV ein Temperaturbolus veranlasst, wobei die dem Temperaturbolus zugrunde liegende Temperaturabweichung mittels der Temperaturbeeinflussungsmittel des zweiten Kreislaufs der EKBV hervorgerufen wird, wobei der erste Kreislauf der EKBV mit dem zweiten Kreislauf der EKBV über einen Wärmetauscher thermisch verbunden ist. In einem zweiten Schritt wird mittels eines ersten Temperatursensors TS2_{auf} stromaufwärts des Wärmetauschers eine Temperatur T2_{auf} im zweiten Kreislauf der EKBV erfasst, während in nachfolgenden dritten Schritt eine Temperatur T2_{ab} im zweiten Kreislauf der EKBV mittels eines stromabwärts des Wärmetauschers angeordneten Temperatursensors TS2_{ab} und eine Temperatur T1_{ab} im abführenden Schenkel des ersten Kreislaufs der EKBV mittels eines stromabwärts des Wärmetauschers angeordneten Temperatursensors TS1_{ab} erfasst werden. In einem weiteren, vierten Schritt erfolgt das Bestimmen eines Indikators der EKBV-Funktion durch in-Beziehung-Setzen der aus den von Temperatursensor TS2_{ab} und Temperatursensor TS1_{ab} erfassten Temperaturen T2_{ab} und T1_{ab} ermittelten Thermodilutionsdaten TDK2_{ab} und TDK1_{ab}.

In einer weiteren Implementierung des Verfahrens kann in einem zusätzlichen Schritt das Bestimmen eines weiteren Indikators der EKBV-Funktion durch in-Beziehung-Setzen der aus den von Temperatursensor TS2_{auf} und Temperatursensor TS1_{ab} erfassten Temperaturen T2_{auf} und T1_{ab} ermittelten Thermodilutionsdaten TDK2_{auf} und TDK1_{ab} umfasst sein.

In dem Verfahren kann ein zusätzlicher Schritt umfasst sein, in dem eine weitere Temperatur im ersten Kreislauf erfasst werden kann, und zwar über den im zuführenden Schenkel des ersten Kreislaufs der EKBV stromaufwärts des Wärmetauschers angeordneten Temperatursensor TS1_{auf}, und eine Korrektur des Indikators der EKBV-Funktion mittels eines Korrekturfaktors aus der Beziehung einer TDK2_{auf} und der vom Temperatursensor TS1_{auf} erfassten Temperatur T1_{auf} erfolgen kann.

In einem weiteren Aspekt betrifft die vorliegende Erfindung ein Computersystem, welches dazu eingerichtet ist, mit einer wie vorstehend beschriebenen, extrakorporalen Blutbehandlungsvorrichtung mit Funktionsüberwachungssystem zusammenzuwirken, wobei das Computersystem folgendes aufweist: Verbindungsmittel, um das Computersystem mit den Temperatursensoren TS2_{auf}, TS2_{ab}, TS1_{ab} und den Temperaturbeeinflussungsmitteln zu verbinden, und Zugriffsmittel zum Zugreifen auf ausführbare Befehle, um zu veranlassen, dass das Computersystem Temperaturbeeinflussungsmittel im zweiten Kreislauf der EKBV steuert, um einen Temperaturbolus im zweiten Kreislauf der EKBV zu veranlassen. Die Zugriffsmittel veranlassen weiterhin, dass das Computersystem die an den Temperatursensoren TS2_{auf}, TS2_{ab}, TS1_{ab} erfassten Temperaturen T2_{auf}, T2_{ab}, T1_{ab} jeweils als Funktion der Zeit aufzeichnet und entsprechend Thermodilutionskurven (TDK) ermittelt und auswertet sowie die TDK2_{ab} und die TDK1_{ab} zueinander in Beziehung setzt und aus der Beziehung der TDK2_{ab} und TDK1_{ab} einen Indikator der EKBV-Funktion bestimmt.

In einem weiteren Aspekt betrifft die Erfindung ein nichtflüchtiges, computerlesbares Speichermedium mit computerlesbaren Anweisungen zum Bestimmen eines Indikators der Funktion einer extrakorporalen Blutbehandlungsvorrichtung mit Funktionsüberwachungssystem wie vorstehend beschrieben, wobei die computerlesbaren Anweisungen durch ein Computersystem ausführbar sind, um das Computersystem zu veranlassen, dass es Temperaturbeeinflussungsmittel im zweiten Kreislauf der EKBV steuert, um einen Temperaturbolus im zweiten Kreislauf der EKBV zu veranlassen, dass es die an den Temperatursensoren TS2_{auf}, TS2_{ab}, TS1_{ab} erfassten Temperaturen T2_{auf}, T2_{ab}, T1_{ab} jeweils als Funktion der Zeit aufzeichnet und entsprechend Thermodilutionskurven (TDK) ermittelt und auswertet, und dass es die TDK2_{ab} und die TDK1_{ab} zueinander in Beziehung setzt und aus der Beziehung der TDK2_{ab} und TDK1_{ab} einen Indikator der EKBV-Funktion bestimmt.

Wie hier verwendet, schließt die Singularform der Artikel "ein", "der", "die" und "das" die entsprechenden Mehrzahlformen ein, sofern nichts anders angegeben. Beispielsweise umfasst der Ausdruck "ein Flüssigkeitsreservoir" ein entsprechendes Reservoir oder mehrere Reservoire.

Nachfolgend wird anhand der zugehörigen Zeichnung eine besonders bevorzugte Ausführungsform der vorliegenden Erfindung näher erläutert, auf welche die Erfindung jedoch nicht beschränkt ist. Grundsätzlich kann jede im Rahmen der vorliegenden Anmeldung beschriebene bzw. angedeutete Variante der Erfindung besonders vorteilhaft sein, je nach wirtschaftlichen, technischen und gegebenenfalls medizinischen Bedingungen im Einzelfall. Soweit nichts Gegenteiliges dargelegt ist, bzw. soweit grundsätzlich technisch realisierbar, sind einzelne Merkmale der beschriebenen Ausführungsformen austauschbar oder miteinander sowie mit per se aus dem Stand der Technik bekannten Merkmalen kombinierbar, sofern dies im Rahmen des Gegenstands der angehängten Ansprüche geschieht.

### Kurze Beschreibung der Figur

Die Zeichnung ist rein schematisch, und, aus Gründen der Anschaulichkeit, nicht maßstabsgetreu. Insbesondere können Verhältnisse zwischen den Abmessungen, vor allem Durchmesser, Schlauchlängen und Außenabmessungen von tatsächlichen Ausführungen abweichen. In der Praxis können die Abmessungen anhand der Anforderungen im Einzelfall sowie anhand gängiger Standardteile dimensioniert werden.

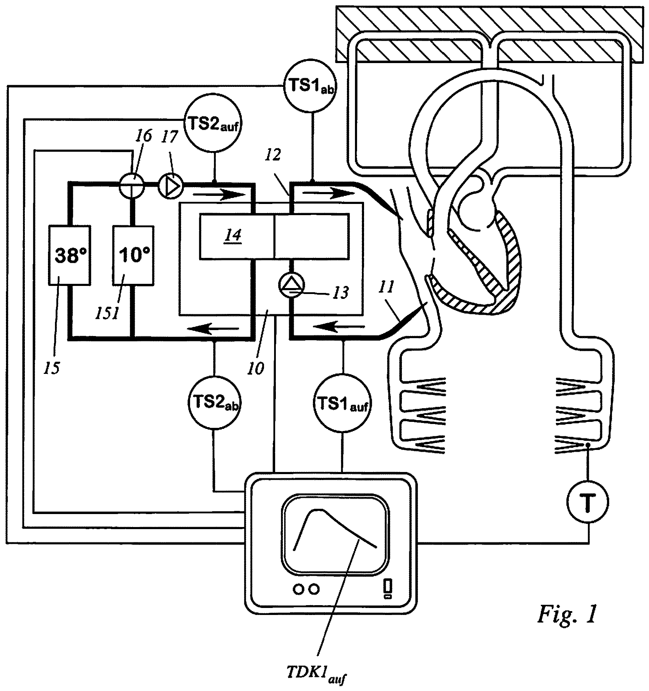

Fig. 1 zeigt eine schematische Übersicht der erfindungsgemäßen extrakorporalen Blutbehandlungsvorrichtung mit Funktionsüberwachungssystem, wobei zur Veranschaulichung die Interaktion mit dem Gefäßsystem eines Patienten dargestellt ist.

### Bevorzugte Ausführung der Erfindung

Fig. 1 zeigt eine schematische Übersicht der erfindungsgemäßen extrakorporalen Blutbehandlungsvorrichtung mit Funktionsüberwachungssystem. Die extrakorporale Blutbehandlungsvorrichtung (EKBV, 10) ist mit dem venösen Gefäßsystem eines Patienten über einen zuführenden Schenkel (11) und einen abführenden Schenkel (12) verbunden. Bei der extrakorporalen Blutbehandlungsvorrichtung kann es sich beispielsweise um eine Vorrichtung zur extrakorporalen Membranoxygenierung (veno-venöse ECMO, vvECMO, wie in Fig. 1 dargestellt) oder um eine Vorrichtung zur Leberdialyse handeln. Der zuführende Schenkel (11) ist beispielsweise über die Femoralvene eingeführt und kommt in der unteren Hohlvene (V. cava inferior) unterhalb der Einmündung der Lebervene zu liegen. Der abführende Schenkel (12) ist beispielsweise über die Jugularvene eingeführt und kommt in der oberen Hohlvene (V. cava superior) unmittelbar vor dem rechten Vorhof zu liegen. Über den zuführenden Schenkel (11) wird der EKBV im Falle der ECMO sauerstoffarmes, venöses Blut zugeführt, während das oxygenierte Blut über den abführenden Schenkel (12) zum rechten Vorhof gelangt. Die erfindungsgemäße EKBV kann in der Form einer ECMO ebenfalls in Situationen angewendet werden, bei welcher das deoxygenierte Blut über die Femoralvene zur ECMO geleitet und das oxygenierte Blut über die Femoralarterie in das Gefäßsystem des Patienten zurückgeleitet wird (vaECMO); in dieser Anordnung kann die ECMO zusätzlich zum Vorrichtungskreislauf die gesamte Pumpleistung im Patientenkreislauf übernehmen. Das Patientenblut wird zur Behandlung mittels einer Pumpe (13) über eine Membran geleitet, beispielsweise eine Hämodialysemembran, eine Hämofiltrationsmembran, eine Hämodiafiltrationsmembran oder eine Oxygenatormembran einer ECMO. Zuführender und abführender Schenkel, sowie die mit der eigentlichen Blutbehandlungsvorrichtung (Membran) verbundenen Leitungen bilden einen ersten Kreislauf aus. Die vorzugsweise als Zentrifugalpumpe ausgebildete Pumpe (13) wird dazu verwendet, die Zirkulation des Bluts durch den ersten Kreislauf zu steuern und zu regulieren. Die in einem extrakorporalen Kreislauf herkömmlicher Blutbehandlungsvorrichtungen verwendeten Pumpen sind in der Regel mit einem Steuerungsgerät verbunden und können so direkt gesteuert werden, sodass diese Pumpen entweder eine relativ konstante Flussrate durch den extrakorporalen Kreislauf und die eigentliche Blutbehandlungsvorrichtung (Membran) oder eine variable Flussrate bereitstellen können. In der erfindungsgemäßen EKBV mit Funktionsüberwachungssystem ist neben dem ersten Kreislauf, welcher dem extrakorporalen Kreislauf einer herkömmlichen EKBV entspricht, ein zweiter Kreislauf vorgesehen, der mit dem ersten Kreislauf der EKBV über einen Wärmetauscher (14) thermisch verbunden ist und Temperaturbeeinflussungsmittel (15) aufweist. Die Übertragung thermischer Energie findet über die wärmedurchlässige Wand des Wärmetauschers statt, welche den ersten und den zweiten Kreislauf voneinander trennt (indirekter Wärmeaustausch). In der gezeigten Ausführungsform arbeitet der Wärmeaustauscher (14) im Gegenstromprinzip, ebenso bevorzugt ist aber auch die Arbeitsweise mit diffuser Stromrichtung. Die Temperaturbeeinflussungsmittel (15) sind dazu eingerichtet, die Temperatur der Flüssigkeit im zweiten Kreislauf zu beeinflussen, um über Wärmefluss (Wärmetauscher) die Temperatur im ersten Kreislauf (Patientenblut) zu beeinflussen. Die Temperaturbeeinflussungsmittel können eine Heizeinrichtung aufweisen, welche dazu eingerichtet ist, dem zweiten Kreislauf thermische Energie in Form von Wärme im zuzuführen; alternativ können die Temperaturbeeinflussungsmittel auch eine Kühleinrichtung aufweisen, welche dazu eingerichtet ist, dem zweiten Kreislauf thermische Energie zu entziehen. In der dargestellten Ausführungsform verwenden die Temperaturbeeinflussungsmittel (15) eine Flüssigkeit, vorzugsweise Wasser, zum Erhitzen/Kühlen der Flüssigkeit des zweiten Kreislaufs; wie dargestellt, kann ein mit kaltem Wasser gefülltes Reservoir (151) über Schaltmittel (16) dem zweiten Kreislauf zugeschaltet werden. Eine im zweiten Kreislauf angeordnete zweite Pumpe (17) ist dazu eingerichtet, die Flüssigkeit im zweiten Kreislauf zu zirkulieren. Die erfindungsgemäße EKBV umfasst ein Funktionsüberwachungssystem zur Bestimmung eines Indikators der EKBV Funktion. Insbesondere eignet sich das System zur Überwachung der Funktion der Membran, über welche die Blutbehandlung erfolgt. Das Funktionsüberwachungssystem weist einen im zweiten Kreislauf stromaufwärts des Wärmetauschers (14) angeordneten Temperatursensor TS2_{auf} sowie einen im zweiten Kreislauf stromabwärts des Wärmetauschers (14) angeordneten Temperatursensor TS2_{ab}. Zum Funktionsüberwachungssystem gehört ferner ein im ersten Kreislauf stromabwärts des Wärmetauschers angeordneten Temperatursensor TS1_{ab}. Die Begriffe "stromabwärts" und "stromaufwärts" beziehen sich dabei auf die jeweiligen Flussrichtungen des ersten und zweiten Kreislaufs im Wärmetauscher. Wenn der Wärmetauscher im Gegenstromprinzip arbeitet, sind die Flussrichtungen der Flüssigkeiten im ersten und zweiten Kreislauf gegenläufig, wenn der Wärmetauscher im Gleichstromprinzip arbeitet, sind die Flussrichtungen der Flüssigkeiten im ersten und zweiten Kreislauf gleichgerichtet. Bei einem mit ungerichteter Strömung arbeitenden Wärmetauscher sind ebenfalls ein stromaufwärts liegende Eingang in den Wärmetauscher und ein stromabwärts liegender Ausgang aus diesem heraus vorhanden. In der vorliegenden Ausführungsform ist im zuführenden Schenkel (11) des ersten Kreislaufs stromaufwärts des Wärmetauschers (14) der EKBV der Temperatursensor TS1_{auf} angeordnet. Das mit den Temperatursensoren TS1_{ab}, TS1_{auf}, TS2_{ab}, TS2_{auf} verbundene Computersystem (40) ist dazu ausgelegt, mittels der Temperaturbeeinflussungsmittel (15) einen Temperaturbolus im zweiten Kreislauf der EKBV zu veranlassen. Zweckmäßigerweise ist das Computersystem (40) mit den Schaltmitteln (16) und der Pumpe (17) des zweiten Kreislaufs verbunden. Insbesondere kann das Computersystem die Pumpengeschwindigkeit zu Erzeugung eines weitgehend steilen Temperaturbolus hinsichtlich des Wärmetauschers anpassen. Aus den von den Temperatursensoren TS1_{ab}, TS1_{auf}, TS2_{ab}, TS2_{auf} erfassten Temperaturen T1_{ab}, T1_{auf}, T2_{ab}, T2_{auf} kann mittels des Computersystems auf bekannte Art und Weise jeweils ein Temperaturverlauf als Funktion der Zeit aufgezeichnet und in einer jeweiligen Thermodilutionskurve ausgewertet werden. Weiterhin ist das Computersystem dazu ausgelegt, aus den ermittelten Thermodilutionskurven (TDK) die TDK_{2ab} und die TDK_{1ab} zueinander in Beziehung zu setzen und aus der Beziehung der TDK2_{ab} und der TDK_{1ab} einen Indikator der EKBV-Funktion zu bestimmen. Weiterhin kann das Computersystem dazu ausgelegt sein, aus den ermittelten Thermodilutionskurven (TDK) die TDK2_{auf} und die TDK_{1ab} zueinander in Beziehung zu setzen und aus der Beziehung der TDK2_{auf} und der TDK_{1ab} einen weiteren Indikator der EKBV-Funktion zu bestimmen. Dieser jeweilige Indikator kann mittels eines Korrekturfaktors aus der Beziehung der TDK2_{auf} und der von Temperatursensor TS1_{auf} erfassten Temperatur T1_{auf} korrigiert werden, indem aus dem Verhältnis TDK2_{aud}/TDK1_{auf} der anfängliche Temperaturgradient zwischen den beiden Kreisläufen abgeleitet und zur Korrektur der Beziehung TDK2_{ab}/TDK1_{ab} berücksichtigt wird.

Wie in Fig. 1 dargestellt, erlaubt die bevorzugte Ausführungsformen auch die Erfassung einer der extrakorporalen Blutbehandlungsvorrichtung zuzuordnenden Temperaturabweichung, indem der vom zusätzlichen Temperatursensor TS1ₐᵤᵣ gemessene Temperaturwert T1_{auf} zu der aus den Werten des Temperatursensors TS1_{ab} generierten TDK1_{ab} in Beziehung gesetzt wird.

### Bezugszeichenliste

- 10: extrakorporale Blutbehandlungsvorrichtung, EKBV
- 11: zuführender Schenkel der EKBV
- 12: abführender Schenkel der EKBV
- 13: Pumpe im ersten Kreislauf
- 14: Wärmetauscher
- 15: Temperaturbeeinflussungsmittel
- 151: Flüssigkeitsreservoir, Temperaturbeeinflussungsmittel
- 16: Schaltmittel
- 17: Pumpe im zweiten Kreislauf

### Literatur:

Dornia, C. e al., (2015), Coagulation Activity During Venovenous ECMO Therapy. Artificial Organs, 39: 782-787.

## Patentansprüche

1. Extrakorporale Blutbehandlungsvorrichtung (EKBV, 10) mit Funktionsüberwachungssystem, wobei die EKBV zur Verbindung mit dem Gefäßsystem eines Patienten einen zuführenden (11) und einen abführenden Schenkel (12) aufweist, wobei die EKBV (10) in einem ersten Kreislauf mindestens eine zwischen dem zuführenden (11) und dem abführenden Schenkel (12) angeordnete erste Pumpe (13) zur Bewegung von Patientenblut und in einem zweiten, mit dem ersten Kreislauf der EKBV über einen Wärmetauscher (14) thermisch verbundenen, flüssigkeitsgefüllten Kreislauf Temperaturbeeinflussungsmittel (15) aufweist,
wobei das Funktionsüberwachungssystem folgendes aufweist:
a. einen im zweiten Kreislauf stromaufwärts des Wärmetauschers (14) angeordneten Temperatursensor TS2_{auf} und einen im zweiten Kreislauf stromabwärts des Wärmetauschers (14 ) angeordneten Temperatursensor TS2_{ab},
b. einen im abführenden Schenkel (12) des ersten Kreislaufs der EKBV (10) stromabwärts des Wärmetauschers (14) angeordneten Temperatursensor TS1_{ab},
c. ein Computersystem (40), welches mit den Temperatursensoren (TS2_{auf}, TS2_{ab}, TS1_{ab}) und den Temperaturbeeinflussungsmitteln (15) verbunden ist, und dazu ausgelegt ist, mittels der Temperaturbeeinflussungsmittel (15) einen Temperaturbolus im zweiten Kreislauf der EBKV (10) zu veranlassen, die an den Temperatursensoren TS2_{auf}, TS2_{ab}, TS1_{ab} erfassten Temperaturen T2_{auf}, T2_{ab}, T1_{ab} jeweils als Funktion der Zeit aufzuzeichnen und entsprechend Thermodilutionskurven (TDK) zu ermitteln und auszuwerten, und weiterhin dazu ausgelegt ist, die TDK2_{ab} und die TDK1_{ab} zueinander in Beziehung zu setzen und aus der Beziehung der TDK2_{ab} und TDK1_{ab} einen Indikator der EKBV-Funktion zu bestimmen.

2. Vorrichtung gemäß Anspruch 1, wobei das Computersystem dazu ausgelegt ist, die TDK2_{auf} und die TDK1_{ab} zueinander in Beziehung zu setzen und aus der Beziehung der TDK2_{auf} und TDK1_{ab} einen weiteren Indikator der EKBV-Funktion zu bestimmen.

3. Vorrichtung gemäß einem der Ansprüche 1 oder 2, wobei das Funktionsüberwachungssystem weiterhin aufweist einen im zuführenden Schenkel (11) des ersten Kreislaufs stromaufwärts des Wärmetauschers (14) der EKBV (10) angeordneten Temperatursensor TS1_{auf}, wobei der Indikator der EKBV-Funktion mittels eines Korrekturfaktors aus der Beziehung der TDK2_{auf} und der von Temperatursensor TS1_{auf} erfassten Temperatur T1_{auf} korrigiert wird.

4. Vorrichtung gemäß einem der vorhergehenden Ansprüche, wobei das Computersystem (40) dazu ausgelegt ist, mindestens eine zweite, mit den Temperaturbeeinflussungsmitteln verbundene Pumpe (17) im zweiten Kreislauf anzusteuern, derart, dass die Pumpengeschwindigkeit zur Erzeugung einer weitgehend steilen Temperaturdifferenz angepasst ist.

5. Vorrichtung gemäß Anspruch 4, wobei die zweite Pumpe (17) im zweiten Kreislauf stromaufwärts des Wärmetauschers (14) angeordnet ist.

6. Vorrichtung gemäß einem der Ansprüche 1 bis 3, wobei die Temperaturbeeinflussungsmittel (15) im ersten Kreislauf der EKBV (10) einen Temperaturbolus erzeugen.

7. Vorrichtung gemäß einem der vorhergehenden Ansprüche, wobei die Temperaturbeeinflussungsmittel (15) stromabwärts der ersten Pumpe (13) angeordnet sind.

8. Vorrichtung gemäß einem der vorhergehenden Ansprüche, wobei die Temperaturbeeinflussungsmittel (15) Schaltmittel (16) zum Schalten zwischen mindestens zwei Temperaturen umfassen.

9. Vorrichtung gemäß Anspruch 8, wobei die Schaltmittel (16) zwischen mindestens zwei unterschiedlich temperierten Flüssigkeitsreservoirs (151) schalten.

10. Vorrichtung gemäß einem der vorhergehenden Ansprüche, wobei die EKBV (10) eine Vorrichtung zur extrakorporalen Membranoxygenierung ist.

11. Vorrichtung gemäß Anspruch 10, wobei die Temperaturbeeinflussungsmittel (15) im Bereich des Oxygenators der ECMO angeordnet sind.

12. Vorrichtung gemäß einem der vorhergehenden Ansprüche, wobei die Temperaturbeeinflussungsmittel (15) extern an eine Heizeinheit der EKBV angeschlossen werden.

13. Computersystem (40), welches dazu eingerichtet ist, mit einer extrakorporalen Blutbehandlungsvorrichtung mit Funktionsüberwachungssystem gemäß einem der Ansprüche 1-12 zusammenzuwirken,
wobei das Computersystem folgendes aufweist:
Verbindungsmittel, um das Computersystem mit den Temperatursensoren TS2_{auf}, TS2_{ab}, TS1_{ab} und den Temperaturbeeinflussungsmitteln zu verbinden, und Zugriffsmittel zum Zugreifen auf ausführbare Befehle, um zu veranlassen, dass das Computersystem
a. Temperaturbeeinflussungsmittel im zweiten Kreislauf der EKBV steuert, um einen Temperaturbolus im zweiten Kreislauf der EKBV zu veranlassen,
b. die an den Temperatursensoren TS2_{auf}, TS2_{ab}, TS1_{ab} erfassten Temperaturen T2_{auf}, T2_{ab}, T1_{ab} jeweils als Funktion der Zeit aufzeichnet und entsprechend Thermodilutionskurven (TDK) ermittelt und auswertet, und
c. die TDK2_{ab} und die TDK1_{ab} zueinander in Beziehung setzt und aus der Beziehung der TDK2_{ab} und TDK1_{ab} einen Indikator der EKBV-Funktion bestimmt.

14. Nichtflüchtiges, computerlesbares Speichermedium mit computerlesbaren Anweisungen zum Bestimmen eines Indikators der Funktion einer extrakorporalen Blutbehandlungsvorrichtung mit Funktionsüberwachungssystem gemäß einem der Ansprüche 1-11, wobei die computerlesbaren Anweisungen durch ein Computersystem ausführbar sind, um das Computersystem zu veranlassen, dass es
a. Temperaturbeeinflussungsmittel im zweiten Kreislauf der EKBV steuert, um einen Temperaturbolus im zweiten Kreislauf der EKBV zu veranlassen,
b. die an den Temperatursensoren TS2_{auf}, TS2_{ab}, TS1_{ab} erfassten Temperaturen T2_{auf}, T2_{ab}, T1_{ab} jeweils als Funktion der Zeit aufzeichnet und entsprechend Thermodilutionskurven (TDK) ermittelt und auswertet, und
c. die TDK2_{ab} und die TDK1_{ab} zueinander in Beziehung setzt und aus der Beziehung der TDK2_{ab} und TDK1_{ab} einen Indikator der EKBV-Funktion bestimmt.

## Claims

1. Extracorporeal blood treatment device (EKBV, 10) having a function-monitoring system, wherein the EKBV comprises an afferent line (11) and an efferent line (12) for connecting to the vascular system of a patient, wherein in a first circuit the EKBV (10) has at least one first pump (13) arranged between the afferent line (11) and the efferent line (12) for moving patient's blood, and comprises temperature-influencing means (15) in a second, liquid-filled circuit that is thermally connected to the first circuit of the EKBV by a heat exchanger (14),
wherein the function-monitoring system comprises the following:
a. a temperature sensor TS2_{auf} arranged in the second circuit upstream of the heat exchanger (14) and a temperature sensor TS2_{ab} arranged in the second circuit downstream of the heat exchanger (14);
b. a temperature sensor TS1_{ab} arranged in the efferent line (12) of the first circuit of the EKBV (10) downstream of the heat exchanger (14);
c. a computer system (40) connected to the temperature sensors (TS2_{auf}, TS2_{ab}, TS1_{ab}) and the temperature influencing means (15), and configured to use the temperature influencing means (15) to induce a temperature bolus in the second circuit of the EKBV (10), to record the temperatures T2_{auf}, TS2_{ab}, TS1_{ab} detected at the temperature sensors TS2_{auf}, TS2_{ab}, TS1_{ab}, respectively, as a function of time and to determine and evaluate corresponding thermodilution curves (TDK), and is furthermore configured to relate TDK2_{ab} and TDK1_{ab} to one another and to determine an indicator of the EKBV function from the relationship of TDK2_{ab} and TDK1_{ab}.

2. Device according to claim 1, wherein the computer system is designed to relate TDK2_{auf} and TDK1_{ab} to one another and to determine a further indicator of the EKBV function from the relationship of TDK2_{auf} and TDK1_{ab}.

3. Device according to one of claims 1 or 2, wherein the function-monitoring system further comprises a temperature sensor TS1_{auf} arranged in the afferent line (11) of the first circuit aufstream of the heat exchangers (14) of the EKBV (10), wherein the indicator of EKBV function is corrected by means of a correction factor from the relationship of TDK2_{auf} and the temperature T1_{auf} detected by temperature sensor TS1_{auf}.

4. Device according to one of the preceding claims, wherein the computer system (40) is configured to control at least one second pump (17) connected to the temperature influencing means in the second circuit such that the pump speed is adjusted to generate a substantially steep temperature difference.

5. Device according to claim 4, wherein the second pump (17) is arranged in the second circuit upstream of the heat exchanger (14).

6. Device according to one of claims 1 to 3, wherein the temperature influencing means (15) generate a temperature bolus in the first circuit of the EKBV (10).

7. Device according to one of the preceding claims, wherein the temperature influencing means (15) are arranged downstream of the first pump (13).

8. Device according to one of the preceding claims, wherein the temperature influencing means (15) comprise switching means (16) for switching between at least two temperatures.

9. Device according to claim 8, wherein the switching means (16) switch between at least two different temperature-controlled liquid reservoirs (151).

10. Device according to one of the preceding claims, wherein the EKBV (10) is a device for extracorporeal membrane oxygenation.

11. Device according to claim 10, wherein the temperature influencing means (15) are arranged in the region of the oxygenator of the ECMO.

12. Device according to one of the preceding claims, wherein the temperature influencing means (15) are connected externally to a heating unit of the EKBV.

13. Computer system (40) configured to interact with an extracorporeal blood treatment device having a function-monitoring system according to one of claims 1-12, wherein the computer system comprises the following:
Connection means for connecting the computer system to the temperature sensors TS2_{auf}, TS2_{ab}, TS1_{ab}, and the temperature influencing means, and access means for accessing executable commands to cause the computer system:
a. to control temperature influencing means in the second circuit of the EKBV in order to induce a temperature bolus in the second circuit of the EKBV;
b. to record the temperatures T2_{auf}, T2_{ab}, T1_{ab} detected at the temperature sensors TS2_{auf}, TS2_{ab}, TS1_{ab}, in each case as a function of time, and accordingly to detect and evaluate to thermodilution (TDK); and,
c. to relate TDK2_{ab} and TDK1_{ab} to one another and determine an indicator of the EKBV function from the relationship of TDK2_{ab} and TDK1_{ab}.

14. Non-volatile, computer-readable storage medium with computer-readable instructions for determining an indicator of the function of an extracorporeal blood treatment device having a function-monitoring system according to any one of claims 1-11, wherein the computer-readable instructions are executable by a computer system to cause the computer system:
a. to control temperature influencing means in the second circuit of the EKBV to induce a temperature bolus in the second cycle of the EKBV;
b. to record the temperatures T2_{auf}, T2_{ab}, T1_{ab} detected on the temperature sensors TS2_{auf}, TS2_{ab}, TS1_{ab}, in each case as a function of time, and to determine and evaluate thermodilution curves (TDK) accordingly; and,
c. to relate the TDK2_{ab} and the TDK1_{ab} to one another and to determine an indicator of the EKBV function from the relationship of TDK2_{ab} and TDK1_{ab}.

## Revendications

1. Dispositif de traitement sanguin extracorporel (EKBV, 10) avec un système de surveillance du fonctionnement, dans lequel l'EKBV comprend, pour la liaison avec un système vasculaire d'un patient, une branche d'alimentation (11) et une branche d'évacuation (12), dans lequel l'EKBV (10) comprend, dans un premier circuit, au moins une première pompe (13), disposée entre la branche d'alimentation (11) et la branche d'évacuation (12), pour le déplacement du sang du patient et, dans un deuxième circuit rempli de liquide, relié thermiquement, par l'intermédiaire d'un échangeur thermique (14), avec le premier circuit de l'EKBV, des moyens de contrôle de la température (15),
dans lequel le système de surveillance de fonctionnement comprend ce qui suit : :
a. un capteur de température TS2_{auf} disposé dans le deuxième circuit en amont de l'échangeur thermique (14) et un capteur de température TS2_{ab} disposé dans le deuxième circuit en aval de l'échangeur thermique (14),
b. un capteur de température TS1_{ab} disposé dans la branche d'évacuation (12) du premier circuit de l'EKBV (10) en aval de l'échangeur thermique (14),
c. un système informatique (40) qui est relié avec les capteurs de température (TS2_{auf}, TS2_{ab}, TS1_{ab}) et les moyens de contrôle de la température (15) et qui est conçu pour permettre, à l'aide des moyens de contrôle de la température (15), un bolus de température dans le deuxième circuit de l'EKBV (10), pour enregistrer les températures T2_{auf}, T2_{ab}, T1_{ab}, mesurées au niveau des capteurs de température TS2_{auf}, TS2_{ab}, TS1_{ab}, chacune en fonction du temps et pour les déterminer et les analyser en fonction de courbes de thermodilution (TDK) et qui est en outre conçu pour mettre en relation la TDK2_{ab} et la TDK1_{ab} entre elles et pour déterminer, à partir de la relation entre la TDK2_{ab} et la TDK1_{ab}, un indicateur du fonctionnement de l'EKBV.

2. Dispositif selon la revendication 1, dans lequel le système informatique est conçu pour mettre en relation la TDK2_{auf} et la TDK1_{ab} entre elles et pour déterminer, à partir de la relation entre la TDK2_{auf} et la TDK1_{ab}, un autre indicateur du fonctionnement de l'EKBV.

3. Dispositif selon l'une des revendications 1 ou 2, dans lequel le système de surveillance du fonctionnement comprend en outre un capteur de température TS1_{auf}, disposé dans la branche d'alimentation (11) du premier circuit, en amont de l'échangeur thermique (14) de l'EKBV (10), dans lequel l'indicateur du fonctionnement de l'EKBV est corrigé au moyen d'un facteur de correction à partir de la relation entre la TDK2_{auf} et la température T1_{auf} mesurée par le capteur de température TS1_{auf}.

4. Dispositif selon l'une des revendications précédentes, dans lequel le système informatique (40) est conçu pour contrôler au moins une deuxième pompe (17) reliée avec les moyens de contrôle de température dans le deuxième circuit, de sorte que la vitesse de pompage est adaptée pour la production d'une différence de température largement pentue.

5. Dispositif selon la revendication 4, dans lequel la deuxième pompe (17) est disposée dans le deuxième circuit en amont de l'échangeur thermique (14).

6. Dispositif selon l'une des revendications 1 à 3, dans lequel les moyens de contrôle de la température (15) produisent, dans le premier circuit de l'EKBV (10), un bolus de température.

7. Dispositif selon l'une des revendications précédentes, dans lequel les moyens de contrôle de la température (15) sont disposés en aval de la première pompe (13).

8. Dispositif selon l'une des revendications précédentes, dans lequel les moyens de contrôle de la température (15) comprennent des moyens de commutation (16) pour la commutation entre au moins deux températures.

9. Dispositif selon la revendication 8, dans lequel les moyens de commutation (16) commutent entre au moins deux réservoirs de liquide (151) tempérés différemment.

10. Dispositif selon l'une des revendications précédentes, dans lequel l'EKBV (10) est un dispositif pour l'oxygénation par membrane extracorporelle.

11. Dispositif selon la revendication 10, dans lequel les moyens de contrôle de la température (15) sont disposés au niveau de l'oxygénateur de l'ECMO.

12. Dispositif selon l'une des revendications précédentes, dans lequel les moyens de contrôle de la température (15) sont raccordés à l'extérieur à une unité de chauffage de l'EKBV.

13. Système informatique (40) qui est conçu pour interagir avec un dispositif de traitement sanguin extracorporel avec système de surveillance du fonctionnement selon l'une des revendications 1 à 12,
dans lequel le système informatique comprend ce qui suit :
des moyens de liaison afin de relier le système informatique avec les capteurs de température TS2_{auf}, TS2_{ab}, TS1_{ab} et les moyens de contrôle de la température,
et des moyens d'accès pour l'accès à des instructions à exécuter, afin de faire en sorte que l'ordinateur
a. contrôle des moyens de contrôle de la température dans le deuxième circuit de l'EKBV, afin de produire un bolus de température dans le deuxième circuit de l'EKBV,
b. enregistre les températures T2_{auf}, T2_{ab}, T1ₐₚ, mesurées au niveau des capteurs de température TS2_{auf}, TS2_{ab}, TS1_{ab}, chacune en fonction du temps et les détermine et les analyse en fonction de courbes de thermodilution (TDK) et
c. met en relation la TDK2_{ab} et la TDK1_{ab} entre elles et détermine, à partir de la relation entre la TDK2_{ab} et la TDK1_{ab}, un indicateur du fonctionnement de l'EKBV.

14. Support de stockage non volatil lisible par un ordinateur, avec des instructions lisibles par un ordinateur pour la détermination d'un indicateur du fonctionnement d'un dispositif de traitement sanguin extracorporel avec système de surveillance du fonctionnement selon l'une des revendications 1 à 11, dans lequel les instructions lisibles par un ordinateur peuvent être exécutées afin de faire en sorte que le système informatique
a. contrôle des moyens de contrôle de la température dans le deuxième circuit de l'EKBV, afin de produire un bolus de température dans le deuxième circuit de l'EKBV,
b. enregistre les températures T2_{auf}, T2_{ab}, T1_{ab}, mesurées au niveau des capteurs de température TS2_{auf}, TS2_{ab}, TS1_{ab}, chacune en fonction du temps et les détermine et les analyse en fonction de courbes de thermodilution (TDK) et
c. met en relation la TDK2_{ab} et la TDK1_{ab} entre elles et détermine, à partir de la relation entre la TDK2_{ab} et la TDK1_{ab}, un indicateur du fonctionnement de l'EKBV.
